# EUROPEAN PATENT SPECIFICATION

(11) **EP 2 743 259 B1**
(45) Date of publication and mention of the grant of the patent: **27.09.2017**
(21) Application number: 12822488.8
(22) Date of filing: 23.04.2012
(51) Int. Cl.: C07D 207/273

(54) **METHOD FOR PURIFYING (S)-OXIRACETAM**
VERFAHREN ZUR REINIGUNG VON (S)-OXIRACETAM
PROCÉDÉ POUR PURIFIER LE (S)-OXIRACÉTAM

(30) Priority: 11.08.2011 CN 201110229933
(43) Date of publication of application: 18.06.2014
(73) Proprietor: CHONGQING RUZER PHARMACEUTICALS CO., LTD., Yubei District Chongqing 401120 (CN)
(72) Inventor: YE, Lei, Yubei Chongqing 401120 (CN); RONG, Zuyuan, Yubei Chongqing 401120 (CN)
(74) Representative: Harrison, Scott David
(86) International application number: PCT/CN2012/074501
(87) International publication number: WO 2013/020388

(56) References cited:
- EP-A1- 2 573 071
- EP-A1- 2 743 258
- EP-A1- 2 743 260
- CN-A- 101 367 757
- CN-A- 101 575 309
- CN-A- 102 101 836
- CN-A- 102 321 007
- S. BANFI ET AL: "Cyclic GABA-GABOB analogues IV - Activity on learning and memory", FARMACO, EDIZIONE SCIENTIFICA, vol. 39, no. 1, 1984, pages 16-22, XP001008330, ISSN: 0430-0920

## Description

### Field of the Invention

This invention relates to a purification method by crystallization, in particular, relates to a purification method of (S)-4-hydroxy-2-oxo-1-pyrrolidine acetamide by crystallization.

### Description of the Prior art

Oxiracetam is a nootropic drug, which is firstly synthesized by Italy Smith Kline Beecham Ltd in 1974 and came into the market in 1987 in Italy. (S)-oxiracetam is a single enantiomer of oxiracetam, the chemical name of which is (S)-4-hydroxy-2-oxo-1-pyrrolidine acetamide (hereinafter referred to as (S)-oxiracetam for short).

Oxiracetam can promote the synthesis of phosphoryl choline and phosphoryl ethanolamine, and promote cerebral metabolism, shows stimulatory effects to specific central nervous pathway through blood-brain barrier, increases the ATP/ADP ratio in the brain and increases the synthesis of protein and nucleic acid in the brain, can improve learning and memory functions of patients with intelligence disorder, and the drug itself have no direct vascular activity, nor the central excitation, its influence on learning and memory abilities is a persistent promoting effect.

CN1513836, CN1948285 and CN101121688 respectively reports a process for synthesis of racemate consisting of two isomers (S)-oxiracetam and (R)-oxiracetam; CN101367757 and CN101575309 respectively reports a method of the preparation of (S)-oxiracetam; CN1424034, CN1555794, CN1562000 and CN101152175 respectively reports a method of the preparation of a injection formulation of oxiracetam, a dispersible tablet of oxiracetam, a lyophilized formulation of oxiracetam and a novel formulation of oxiracetam; WO93/06826 discloses that (S)-oxiracetam has good therapeutic effects on the improvement of intelligence. However, currently the process for the purification of (S)-oxiracetam is complicated or the purity of the product is not higher enough, thereby it is difficult to prepare (S)-oxiracetam with high purity under low cost and simple process condition.

### Summary of the Invention

One objective of the invention is to provide a purification method of (S)-oxiracetam. The purification method is simple and low cost, and the (S)-oxiracetam prepared by which contains low impurity and is in high purity.

The objective of the present invention is achieved by the following technical solutions:
A purification method of (S)-oxiracetam, characterized in that:
   (1) dissolving crude (S)-oxiracetam into water to form a solution, wherein the mass volume ratio (g/mL) of the crude (S)-oxiracetam to the water is ranging from 1:0.5 to 1:2, then the obtained solution stands at a temperature of 5 to 15°C for 1 to 3 days to precipitate colorless transparent crystals;
   (2) filtering and washing with ice water of 0 to 5°C;
   (3) vacuum drying to obtain (S)-oxiracetam with high purity.

In order to precipitate the (S)-oxiracetam dissolved in the water sufficiently, the mass volume ratio (g/mL) of crude (S)-oxiracetam to water in step (1) described above is preferably 1:0.8; and the solution stands at a temperature of preferably 11 °C. In order to further improve the purity of (S)-oxiracetam obtained ultimately, the temperature of the ice water used in step (2) described above is 2 to 4°C, and further preferably 3°C; the mass volume ratio (g/mL) of precipitated crystals to ice water in step (2) described above is preferably 1:1 to 1:2. Further preferably, the vacuum drying in step (3) described above is carried out at a temperature of 26 to 28°C for 4 to 5 hours.

Specifically, a purification method of (S)-oxiracetam is performed by the following steps:
(1) dissolving crude (S)-oxiracetam (purity≤89%) in water to form a solution, then the obtained solution stands at 11°C for 26 to 28 hours to precipitate colorless transparent crystals; the mass volume ratio (g/mL) of the crude (S)-oxiracetam to water is 1:0.8;
(2) filtering obtained colorless transparent crystals described above, washing with ice water; the mass volume ratio (g/mL) of the precipitated crystals obtained to ice water is ranging from 1:1 to 1:2, the temperature of the ice water is 3°C;
(3) then vacuum drying at a temperature of 26 to 28°C for 4 to 5 hours to obtain (S)-oxiracetam with high purity.

The present invention has the following advantages:
It is not difficult for a person skilled in the art to know that (S)-oxiracetam has good water solubility and can dissolve into water rapidly to form a solution, therefore people always consider it is impracticable that purifying (S)-oxiracetam by using water as solvent. With conventional ideas, the inventor has adopted organic solvents to purify (S)-oxiracetam, and conducted numerous experimental studies to continually explore various steps and process conditions, however the purity of (S)-oxiracetam obtained finally cannot meet the desirable requirement. The inventor discovered accidentally that in one of the long-term experiments, adopting a purification process using water as solvent can effectively improve the purity of crude (S)-oxiracetam from 89% to 98-98.4% of HPLC purity, which greatly improves the purity of (S)-oxiracetam; meanwhile the method of the invention is simple with mild controlling conditions, low cost, environmentally friendly without the pollution caused by organic solvent, and is very suitable for large-scale manufacture.

### Detailed Description of the Preferred Embodiments

### Example 1

A purification method of (S)-oxiracetam, was performed by the following steps:
1g of crude (S)-oxiracetam (89% purity) was dissolved in 0.8 mL of water, and the obtained solution was left to stand at 11°C for 28 hours to precipitate colorless transparent crystals. The colorless transparent crystals were filtered, washed with 1mL of ice water of 3°C, and dried under vacuum at a temperature of 26∼28°C for 4∼5 hours to obtain 0.7g of colorless crystalline product in HPLC purity of 98.4%.

The preparation of the crude (S)-oxiracetam described above was performed by the following steps:
(a) 28.50g of glycinamide hydrochloride, 20.65g of sodium bicarbonate and 200ml of anhydrous ethanol were charged into a three-necked round-bottom flask to give a mixture, and the pH value was maintained at about 7.4. Then the mixture was heated to reflux with stirring;
(b) After refluxed for 2 hours, 39.08g of ethyl (S) 4-chloro-3-hydroxybutyrate was added dropwise. During the process of the addition, another 20.65g of sodium bicarbonate was added in five portions, and the amount of the base added in each portion was controlled by detecting the pH value of the reaction mixture to maintain it ≤ 8.5;
(c) After the addition of ethyl (S) 4-chloro-3-hydroxybutyrate is completed, the reaction mixture was further refluxed for 24 hours. The reaction was terminated until the content of (S)-4-hydroxy-2-oxo-1-pyrrolidine acetamide measured by HPLC was 74%. The obtained solution was thermal filtered and concentrated to obtain crude product of (S)-4-hydroxy-2-oxo-1-pyrrolidine acetamide;
(d) The obtained crude product was dissolved in 50mL of water to obtain a solution, the solution obtained was treated with 500mL of 001x7 strong acid styrene type cation exchange resin and the fractions containing products were collected. The collected aqueous solution was neutralized with 201x7 strong base styrene type anion exchange resin. The neutralization was determined as completed until the measured pH value of the solution reached 7.0±0.1.

### Example 2

A purification method of (S)-oxiracetam is performed by the following steps:
16L of water was added to dissolve 8kg of crude (S)-oxiracetam (89% purity), and the solution obtained was left to stand at 15°C for 30 hours to precipitate colorless transparent crystals. The colorless transparent crystals were filtered, washed with 10.5 L of ice water of 4°C, and dried under vacuum at room temperature for 5 hours to obtain 5 kg of colorless crystalline product in a HPLC purity of 98.2%.

### Example 3

A purification method of (S)-oxiracetam, is performed by following steps:
0.6L of water was added to dissolve 1kg of crude (S)-oxiracetam (89% purity), the obtained solution was left to stand at 8°C for 25 hours to precipitate colorless transparent crystals. The colorless transparent crystals were filtered, washed with 0.8 L of ice water of 5°C, and dried under vacuum at 30°C for 4 hours to obtain 0.6 kg of colorless crystalline product in a HPLC purity of 98.3%.

## Claims

1. A purification method of (S)-oxiracetam, **characterized in that**:
(1) Dissolving crude (S)-oxiracetam into water to form a solution, then making the solution obtained to stand at a temperature of 5 to 15°C for 1 to 3 days to precipitate colorless transparent crystals, wherein the mass volume ratio (g/mL) of the crude (S)-oxiracetam to the water is ranging from 1:0.5 to 1:2;
(2) Filtering and washing with ice water at a temperature of 0 to 5°C;
(3) Vacuum drying to obtain pure (S)-oxiracetam with high purity.

2. The purification method according to Claim 1 **characterized in that**: the mass volume ratio (g/mL) of the crude (S)-oxiracetam to the water in step (1) is 1:0.8, and the solution stands at a temperature of 11°C.

3. The purification method according to Claim 1 or 2 **characterized in that**: the temperature of the ice water used in step (2) is 2 to 4°C; the mass volume ratio (g/mL) of the precipitated crystals to the ice water in step (2) is ranging from 1:1 to 1:2.

4. The purification method according to Claim 3 **characterized in that**: the temperature of the ice water used in the step (2) is 3°C.

5. The purification method according to any one of the Claims 1-4 **characterized in that**:
the vacuum drying in step (3) is carried out at a temperature of 26 to 28°C for 4 to 5 hours.

6. The purification method according to Claim 1 comprising steps as follows:
(1) Dissolving the crude (S)-oxiracetam (≤ 89% purity) in water to form a solution, then making the solution obtained to stand at a temperature of 11°C for 26 to 28 hours to precipitate colorless transparent crystals, the mass volume ratio (g/mL) of the crude (S)-oxiracetam to the water is 1:0.8;
(2) Filtering the obtained colorless transparent crystals and washing with ice water; the mass volume ratio (g/mL) of the precipitated colorless transparent crystals to the ice water is ranging from 1:1 to 1:2, and the temperature of the ice water is 3°C;
(3) Then drying under vacuum at a temperature of 26 to 28°C for 4 to 5 hours to obtain (S)-oxiracetam with high purity.

## Patentansprüche

1. Reinigungsverfahren von (S)-oxiracetam, **gekennzeichnet durch**:
(1) Auflösen von rohem (S)-oxiracetam in Wasser, um eine Lösung zu bilden, dann Stehenlassen der erhaltenen Lösung bei einer Temperatur von 5 bis 15°C für 1 bis 3 Tage, um farblose transparente Kristalle auszufällen, wobei das Masse Volumen Verhältnis (g/ml) des rohen (S)-oxiracetam zu Wasser von 1:0.5 bis 1:2 reicht;
(2) Filtern und Waschen mit Eiswasser bei einer Temperatur von 0 bis 5°C;
(3) Vakuumtrocknen um pures (S)-oxiracetam mit hoher Reinheit zu erhalten.

2. Reinigungsverfahren nach Anspruch 1, **gekennzeichnet durch**: das Masse Volumen Verhältnis (g/ml) des rohen (S)-oxiracetam zu Wasser im Schritt (1) ist 1:0.8, und die Lösung wird bei einer Temperatur von 11°C stehen gelassen.

3. Reinigungsverfahren nach Anspruch 1 oder 2, **gekennzeichnet durch**: die Temperatur des im Schritt (2) verwendeten Eiswassers beträgt 2 bis 4°C; das Masse Volumen Verhältnis (g/ml) der ausgefällten Kristalle zum Eiswassers im Schritt (2) reicht von 1:1 bis 1:2.

4. Reinigungsverfahren nach Anspruch 3, **gekennzeichnet durch**: die Temperatur des im Schritt (2) verwendeten Eiswassers ist 3°C.

5. Reinigungsverfahren nach einem der Ansprüche 1-4, **gekennzeichnet durch**: das Vakuumtrocknen in Schritt (3) wird bei einer Temperatur von 26 bis 28°C für 4 bis 5 Stunden durchgeführt.

6. Reinigungsverfahren nach Anspruch 1 aufweisend folgende Schritte:
(1) Auflösen von rohem (S)-oxiracetam (≤ 89% Reinheit) in Wasser, um eine Lösung zu bilden, dann Stehenlassen der erhaltenen Lösung bei einer Temperatur von 11°C für 26 bis 28 Stunden, um farblose transparente Kristalle auszufällen, wobei das Masse Volumen Verhältnis (g/ml) des rohen (S)-oxiracetam zu Wasser von 1:0.8 ist;
(2) Filtern der erhaltenen farblosen transparenten Kristalle und Waschen mit Eiswasser; wobei das Masse Volumen Verhältnis (g/ml) der ausgefällten farblosen transparenten Kristalle zu Eiswasser von 1:1 bis 1:2 reicht, und die Temperatur des Eiswassers 3°C beträgt;
(3) Dann Trocknen unter Vakuum bei einer Temperatur von 26 bis 28°C für 4 bis 5 Stunden, um (S)-oxiracetam mit hoher Reinheit zu erhalten.

## Revendications

1. Procédé de purification de (S)-oxiracétam, **caractérisé par** les étapes consistant à :
(1) dissoudre du (S)-oxiracétam brut dans de l'eau pour former une solution, puis faire en sorte que la solution obtenue soit maintenue à une température de 5 à 15°C pendant 1 à 3 jours pour précipiter des cristaux transparents incolores, dans lequel le rapport de la masse au volume (g/ml) du (S)-oxiracétam brut à l'eau est compris entre 1 : 0,5 et 1 : 2 ;
(2) filtrer et laver avec de l'eau glacée à une température de 0 à 5°C ;
(3) sécher sous vide pour obtenir du (S)-oxiracétam pur avec une pureté élevée.

2. Procédé de purification selon la revendication 1 **caractérisé en ce que** : le rapport de la masse au volume (g/ml) du (S)-oxiracétam brut à l'eau dans l'étape (1) est de 1 : 0,8, et la solution est maintenue à une température de 11°C.

3. Procédé de purification selon la revendication 1 ou 2, **caractérisé en ce que** :
la température de l'eau glacée utilisée dans l'étape (2) est de 2 à 4°C ; le rapport de la masse au volume (g/ml) des cristaux précipités à l'eau glacée dans l'étape (2) est compris entre 1 : 1 et 1 : 2.

4. Procédé de purification selon la revendication 3, **caractérisé en ce que** : la température de l'eau glacée utilisée dans l'étape (2) est de 3°C.

5. Procédé de purification selon l'une quelconque des revendications 1 à 4, **caractérisé en ce que** :
le séchage sous vide dans l'étape (3) est effectué à une température de 26 à 28°C pendant 4 à 5 heures.

6. Procédé de purification selon la revendication 1, comprenant les étapes suivantes consistant à :
(1) dissoudre le (S)-oxiracétam brut (≤ 89% de pureté) dans de l'eau pour former une solution, puis faire en sorte que la solution obtenue soit maintenue à une température de 11 °C pendant 26 à 28 heures pour précipiter des cristaux transparents incolores, le rapport de la masse au volume (g/ml) du (S)-oxiracétam brut à l'eau est de 1 : 0,8 ;
(2) filtrer les cristaux transparents incolores obtenus et les laver avec de l'eau glacée ; le rapport de la masse au volume (g/ml) des cristaux transparents incolores précipités à l'eau glacée varie de 1 : 1 à 1 : 2, et la température de feau glacée est de 3°C ;
(3) puis sécher sous vide à une température de 26 à 28°C pendant 4 à 5 heures pour obtenir du (S)-oxiracétam avec une pureté élevée.
